# EUROPÄISCHE PATENTSCHRIFT

(11) **EP 0 340 160 B1**
(45) Veröffentlichungstag und Bekanntmachung des Hinweises auf die Patenterteilung: **28.04.1993**
(21) Anmeldenummer: 89810232.2
(22) Anmeldetag: 23.03.1989
(51) Int. Cl.: A61B 17/56, A61F 5/02

(54) **Bausatz für Implantate**
Kit for implants
Equipement pour implants

(30) Priorität: 27.04.1988 CH 1577/88
(43) Veröffentlichungstag der Anmeldung: 02.11.1989
(73) Patentinhaber: GEBRÜDER SULZER AKTIENGESELLSCHAFT, CH-8401 Winterthur (CH); ALLO PRO AG, 6340 Baar (CH)
(72) Erfinder: Karpf, Kurt, CH-4718 Holderbank (DE)

(56) Entgegenhaltungen:
- EP-A- 0 140 790
- EP-A- 0 220 736
- WO-A-87/01026
- CH-A- 646 857
- DE-A- 2 603 983
- DE-A- 2 834 891
- FR-A- 2 320 078
- US-A- 4 289 123
- US-A- 4 611 582

## Beschreibung

Die Erfindung betrifft ein als Bausatz ausgeführtes Implantat zur Stabilisierung von Teilbereichen der Wirbelsäule, bestehend aus mindestens zwei, an je einem Wirbel fixierbaren Verankerungselementen, an denen den zu stabilisierenden Wirbelsäulenbereich überbrückende Stabilisierungselemente befestigt sind.

Zur Korrektur von Deformationen der Wirbelsäule sind eine Vielzahl von Verfahren und Vorrichtungen bekannt, wobei für die verschiedenen Bereiche der Wirbelsäule unterschiedliche Instrumente und Implantate eingesetzt werden (siehe z.B.
M. Hackenbroch und A.N.Witt "Orthopädisch-chirurgischer Operationsatlas", Band III, Thieme-Verlag Stuttgart, 1974). Diese Vielfalt führt zu einer grossen Anzahl unterschiedlicher Implantate und Instrumente, die eine grosse Lagerhaltung erfordern.

Aus der CH-PS 646 857 ist ein Spondylodese-Stabilisator bekannt, bei dem mittels Knochenschrauben in benachbarten Wirbeln vier gleiche Laschen fixiert sind, in die als Stabilisatoren je mit zwei gegenläufigen Gewinde versehene Bolzen eingeschraubt sind. Mit diesen, die Lasche starr verbindenden Gewindebolzen werden die beiden Wirbel gegeneinander fixiert, wobei durch Drehen der Gewindebolzen in der einen oder der anderen Richtung der Abstand der Laschen verlängert oder verkürzt werden kann. Die von den Stabilisatoren auf die Wirbel ausgeübten Kräfte werden dabei im wesentlichen punktuell weitergeleitet, was zu örtlichen Belastungsspitzen führt. Sehr ähnliche Konstruktionen sind in der EP-A-0 220 736, sowie in den US-A-4,289,123 und 4,611,582 beschrieben.

Schliesslich ist aus der DE-A 28 34 891 ein ausserhalb des Körpers angeordneter Fixateur bekannt, der mit Hilfe von Knochenschrauben an Wirbeln befestigt wird, um gebrochene Wirbel kurzzeitig, d.h. solange bis die Bruchstücke wieder zusammengewachsen sind, gegeneinander zu fixieren. Zwei in den gleichen Wirbel eingeschraubte Knochenschrauben sind dabei über Klemmhalterungen mit einem T-förmigen Trägerkörper verbunden und, in Längsschlitzen verstellbar, fixiert. Untereinander angeodnete Wirbel werden mit Hilfe von, die zugehörigen Trägerkörper verbindenden Gewindebolzen relativ zueinander ruhiggestellt. Als Langzeit- oder Dauerimplantat ist diese Konstruktion absolut ungeeignet, unter anderem deshalb, weil sie beispielsweise dem Patienten ein Liegen auf dem Rücken nicht ermöglicht.

Aufgabe der Erfindung ist es, ein Implantat-System für die Korrektur von Wirbelsäulen-Teilbereichen zu schaffen, das aus wenigen gleichartigen Grundelementen besteht, die in allen Bereichen der Wirbelsäule verwendet werden können, und von denen die bei einer Stabilisierung der mit Hilfe eines Instrumentes durchgeführten Korrekturen erzeugten Kräfte möglichst grossflächig auf die Wirbel, an denen die Grundelemente fixiert sind, verteilt werden.

Diese Aufgabe wird dadurch gelöst, dass der Bausatz aus unter sich gleichartigen, an Wirbeln anliegenden Verankerungsleisten besteht, die an beiden Enden mit je einer Bohrung für den Durchtritt von Fixierungselementen versehen sind und zwischen den Bohrungen Anschlussstellen für die Befestigung der Stabilisierungselemente und/oder von Korrekturinstrumenten aufweisen, ferner dadurch gekennzeichnet, dass die Anschlussstellen als mit einer Gewindebohrung versehene, muldenartige Vertiefungen ausgebildet sind, in welche entsprechende, erhabene Gegenformen der Stabilisierungselemente einlagerbar sind.

Unabhängig von Art, Form und Grösse der Stabilisierungselemente werden bei dem Bausatz in allen Teilbereichen der Wirbelsäule gleichartige Verankerungsleisten verwendet, die mit Knochenschrauben oder Spreizdübel an Wirbeln befestigt werden, die den zu korrigierenden Bereich oben und unten begrenzen. Da die Fixierungspunkte an einem Wirbel durch die stegartige Konstruktion der an dem Wirbel anliegende Verankerungsleiste untereinander verbunden sind, ergibt sich eine Verteilung der Kräfte auf grössere Bereiche der Wirbelkörper als bei der bekannten Konstruktionen; darüberhinaus gewährleistet die gegenseitige Anpassung der muldenförmigen Vertiefungen und der erhabenen Gegenformen eine stabile Verbindung der Verankerungsleisten mit den Stabilisierungselementen.

Mit Hilfe eines Korrekturinstrumentes, das je an einer der Anschlussstellen der zuvor bereits gesetzten Verankerungsleisten zeitweilig befestigt wird, wird die erforderliche Korrektur durchgeführt. Anschliessend werden an je einer oder mehreren der Anschlussstellen Stabilisierungselemente befestigt, ehe das Korrekturinstrument entfernt wird. Das Vorhandensein von mehreren Anschlussstellen ermöglicht dabei eine gleichzeitig vorhandene Befestigung von Instrument und mindestens einem Stabilisierungselement. Selbstverständlich ist es möglich, die vom Instrument besetzten Anschlussstellen nach dessen Entfernung für die Fixierung eines weiteren Stabilisierungselementes zu benutzen.

Um unterschiedlichen Grössen von Wirbeln Rechnung zu tragen, ist es möglich, in einem Bausatz eine Anzahl von Verankerungsleisten vorzusehen, bei denen die Lage der Anschlussstellen untereinander und relativ zu einer Mittelebene der Verankerungsleiste gleich, der Abstand der Bohrungen für die Fixierungselemente jedoch in Stufen variiert ist.

Weiterhin können einzelne Verankerungsleisten auch für eine Fixation am Uebergang vom letzten Lendenwirbel zum Kreuzbein durch an den Enden mindestens nahezu rechtwinklig wegführende Laschen zu Kreuzbein-Verankerungsleisten erweitert sein.

Im folgenden wird die Erfindung anhand eines Ausführungsbeispiels im Zusammenhang mit der Zeichnung näher erläutert.
- Fig. 1: ist eine Aufsicht auf Fig. 2 von oben und stellt eine Verankerungsleiste dar;
- Fig. 2: gibt eine Aufsicht auf Fig. 1 von oben wieder;
- Fig. 3: schliesslich zeigt schematisch den Einsatz des neuen Bausatzes im Bereich der Lendenwirbel und des Kreuzbeins.

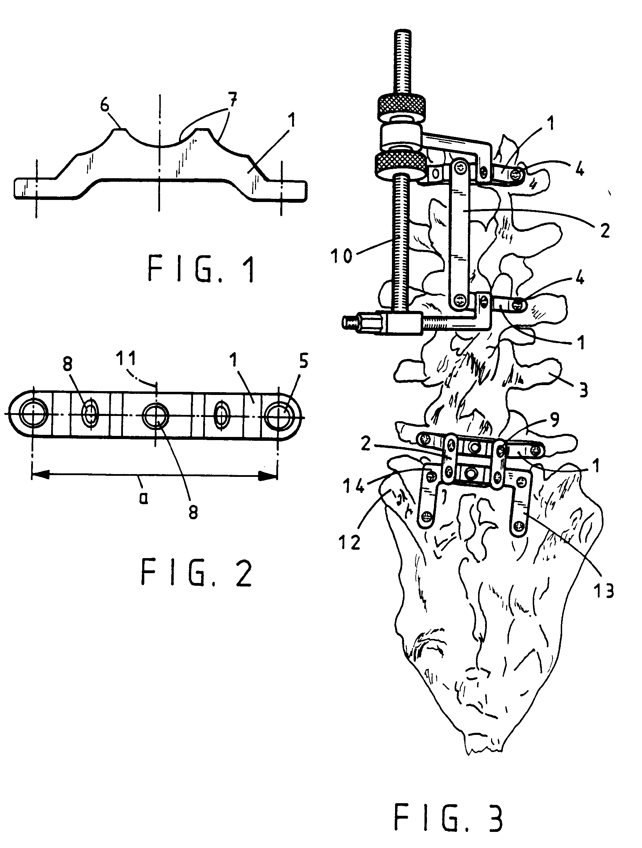

Wie Fig. 3 zeigt, besteht ein Bausatz im wesentlichen aus untereinander gleichen oder mindestens gleichartigen Verankerungsleisten 1 und Stabilisierungselementen 2, die in dem gezeigten Beispiel sich zwischen Wirbeln 3 erstreckende Distraktions- oder Kontraktionsplatten unterschiedlicher Länge sind.

Jede Verankerungsleiste ist ein in seinem Mittelteil brückenartig erhöhter und in seiner Dicke verstärkter Steg (Fig. 1 und 2), der an seinen beiden Enden Bohrungen 5 aufweist. Diese dienen für eine Befestigung der Verankerungsleiste an einem Wirbel 3 mit Hilfe von Knochenschrauben 4 (Fig. 3), die - gegebenenfalls mit Hilfe von Spreizdübel - in dem Wirbelknochen fixiert werden.

Zwischen den Bohrungen 5 ist der Steg mit zahnartigen Erhebungen 6 versehen; zwischen den Erhebungen 6 und diesen und den Bohrungen 5 sind muldenartige Vertiefungen 7 vorhanden, die je eine Gewindebohrung 8 enthalten. In diesen Vertiefungen 7 werden die Stabilisierungselemente 2 gelagert und durch Schrauben 9 (Fig. 3) fixiert. Um die Verbindung zwischen beiden Elementen 1 und 2 zu stabilisieren, sind die Elemente 2 auf ihrer Rückseite mit an die Vertiefungen 7 angepassten "Lagerzapfen" versehen, was nicht ausdrücklich gezeigt ist.

Wie Fig. 3 zeigt, kann in entsprechende Vertiefungen 7 der Verankerungsleisten 1 auch ein Korrekturinstrument 10 befestigt werden, das eine Kontraktion oder Distraktion der "überspannten" Wirbel 3 bewirkt. Ist die Stellung der Wirbel 3 zueinander korrigiert, so kann ohne Behinderung der korrigierte Abstand mit einem oder mehreren Stabilisierungselementen 2 fixiert werden.

Innerhalb eines Bausatzes können alle Verankerungsleisten 1 gleich ausgebildet sein, d.h. gleiche Abstände a der Bohrungen 5 und die gleiche Lagen der Bohrungen 8 relativ zueinander und zu einer Symmetriemittelebene 11 haben. Es ist jedoch auch möglich, die Leisten 1 nur gleichartig auszubilden, wobei vorwiegend die Abstände a der Bohrungen 5 voneinander verschieden sind, um eine Anpassung an unterschiedliche Wirbel-"Grössen" zu ermöglichen. Selbstverständlich kann man auch einige Exemplare der Verankerungsleisten 1 in einem Bausatz vorsehen, die sich nur ähnlich sind, d.h. bei denen die Form gleich zu derjenigen aller übrigen Verankerungsleisten ist, nebem dem Abstand a aber zusätzlich auch noch die Lage der Vertiefungen 7 und/oder Bohrungen 8 variiert ist.

Um den Besonderheiten im Bereich des Kreuzbeins 12 Rechnung zu tragen, kann man schliesslich durch mindestens nahezu senkrecht zu dem Steg der Leiste 1 verlaufende, vorzugsweise in der Nähe der Enden angeordnete Laschen 13 eine oder einige wenige Verankerungsleisten eines Bausatzes zu einer speziellen Kreuzbein-Verankerungsleiste 14 erweitern.

## Patentansprüche

1. Als Bausatz ausgeführtes Implantat zur Stabilisierung von Teilbereichen der Wirbelstäule, bestehend aus mindestens zwei, an je einem Wirbel (3) fixierbaren Verankerungselementen (1), an denen den zu stabilisierenden Wirbelsäulenbereich überbrückende Stabilisierungselemente (2) befestigt sind, dadurch gekennzeichnet, dass der Bausatz aus unter sich gleichartigen, an Wirbeln (3) zur Anlage bringbaren Verankerungsleisten (1) besteht, die an beiden Enden mit je einer Bohrung (5) für den Durchtritt von Fixierungselementen (4) versehen sind und zwischen den Bohrungen (5) Anschlussstellen (7) für die Befestigung der Stabilisierungselemente (2) und/oder von Korrekturinstrumenten (10) aufweisen, ferner dadurch gekennzeichnet, dass die Anschlussstellen (7) als mit einer Gewindebohrung (8) versehene, muldenartige Vertiefungen ausgebildet sind, in welche entsprechende, erhabene Gegenformen der Stabilisierungselemente (2) einlagerbar sind.

2. Implantat nach Anspruch 1, dadurch gekennzeichnet, dass eine Anzahl Verankerungsleisten (1) vorhanden ist, bei denen die Lage der Anschlussstellen (7) untereinander und relativ zu einer Mittelebene der (11) Verankerungsleiste (1) gleich, der Abstand (a) der Bohrungen (5) für die Fixierungselemente (4) jedoch in Stufen variiert ist.

3. Implantat nach Anspruch 1 oder 2, dadurch gekennzeichnet, dass einzelne Verankerungsleisten durch an den Enden mindestens nahezu rechtwinklig wegführende Laschen (13) zu Kreuzbein-Verankerungsleisten (14) erweitert sind.

## Claims

1. An implant in kit form for stabilising parts of the vertebral column and comprising at least two anchoring elements (1) for securing to respective vertebrae (3) and secured to stabilising components (2) which bridge the region of the vertebral column to be stabilised, characterised in that the kit is made up of similar anchoring strips (1) which can be brought to abut the vertebrae (3) and are formed at each end with a bore (5) for receiving securing components (4) and, between the bores (5), have connecting places (7) for securing the stabilising components (2) and/or corrective instruments (10), also characterised in that the connecting places (7) are in the form of trough-like recesses comprising a threaded bore (8) for inserting the stabilising components (2), which have corresponding raised matching shapes.

2. An implant according to claim 1, characterised in that a number of anchoring strips (1) are provided, the position of the connecting places (7) relative to one another and relative to a central plane (11) of the anchoring strip (1) being the same whereas the distance a between the bores (5) for the securing components (4) varies in stages.

3. An implant according to claim 1 or 2, characterised in that individual anchoring strips are widened by tongues (13) extending from their ends at least approximately at right angles, forming sacrum-anchoring strips (14).

## Revendications

1. Module implanté constitué d'un ensemble de composants, destiné à la stabilisation de régions de la colonne vertébrale et consistant en au moins deux éléments d'ancrage (1) se fixant à une vertèbre (3) et auxquels se fixent des éléments de stabilisation (2) qui chevauchent la région de la colonne vertébrale qu'il s'agit de stabiliser, caractérisé en ce que l'ensemble de composants consiste en barrettes semblables d'ancrage (1) se mettant en appui contre des vertèbres (3), comportant à chacune des deux extrémités un trou (5) destiné au passage d'éléments de fixation (4) et comprenant entre les trous (5) des lieux de raccord (7) destinés à la fixation des éléments de stabilisation (2) et/ou d'instruments de correction (10), ledit module implanté étant par ailleurs caractérisé en ce que les lieux de raccord (7) sont conformés en évidements en forme de creux comportant un trou taraudé (8) et dans lesquels se logent les formes correspondantes complémentaires en relief des éléments de stabilisation (2).

2. Module implanté selon la revendication 1, caractérisé en ce qu'il dispose de plusieurs barrettes d'ancrage (1) dans lesquelles la position des lieux de raccord (7) les uns par rapport aux autres et par rapport à un plan de symétrie (11) des barrettes d'ancrage (1) est la même, mais la distance (a) des trous (5) destinés aux éléments de fixation (4) varie par paliers.

3. Module implanté selon la revendication 1 ou 2, caractérisé en ce que certaines barrettes d'ancrage sont complétées en barrettes (14) d'ancrage sur le sacrum par des plaquettes (13) partant au moins à peu près perpendiculairement des extrémités.
